# EUROPEAN PATENT APPLICATION

(11) **EP 1 832 165 A1**
(43) Date of publication of application: **12.09.2007**
(21) Application number: 05809322.0
(22) Date of filing: 24.11.2005
(51) Int. Cl.: A01K 67/033

(54) **FEEDING METHOD AND FEEDING DEVICE FOR RAISING NATURAL ENEMY INSECT AND METHOD OF RAISING NATURAL ENEMY INSECT**

(30) Priority: 26.11.2004 JP 2004343150
(71) Applicant: Kyoto University, Kyoto-shi, Kyoto 606-8501 (JP); Incorporated Administrative Agency, National Agriculture and Bio-Oriented Research Organization, Tsukuba-shi, Ibaraki 305-8517 (JP); Shikoku Research Institute Incorporated, Takamatsu-shi Kagawa 761-0192 (JP)
(72) Inventor: TAKABAYASHI, Junji, (JP); KUGIMIYA, Soichi, (JP); UEFUNE, Masayoshi, (JP); YANO, Eizi, (JP); SHIMODA, Takeshi, (JP); MITSUNAGA, Takayuki, (JP); KANNO, Hiroo, (JP); URANO, Satoru, (JP); UCHIDA, Toru, (JP); KAKIBUCHI, Kazumasa, (JP); OHARA, Yoshitsugu, (JP); NAGASAKA, Kokichi, (JP); ABE, Junichiro, (JP); SANO, Kota, (JP)
(74) Representative: Graf von Stosch, Andreas
(86) International application number: PCT/JP2005/021613
(87) International publication number: WO 2006/057315

(57) **Abstract**

A feeding method and apparatus for breeding natural enemy insects necessary for establishing a farming system that uses less or no pesticides and a rearing method for breeding natural enemy insects are provided.

A feeding apparatus including a food supplying section, a feeding section, and a food introducing section via which the food supplying section and the feeding section are in communication with each other, and an attracting section made of a material having a specific color tone is designed, and is used to breed natural enemy insects.

## Description

### TECHNICAL FIELD

The present invention relates to feeding methods and apparatuses for breeding natural enemy insects that prey on small insect pests harmful to plants, and to rearing methods for breeding natural enemy insects. Specifically, the present invention relates to a feeding method and apparatus for breeding parasitic wasps (e.g., *Cotesia plutellae),* and to a rearing method for breeding natural enemy insects.

### BACKGROUND ART

Conventionally, insecticides (esp., chemically-synthesized insecticides) have been applied in order to control insect pests harmful to crops. However, an excessive application of insecticide poses a danger of giving rise to a new strain of insect pest that has insecticide resistance. It is difficult for insecticides to control garden insect pests whose insecticide resistance has been highly developed.

Further, chemically-synthesized insecticides exert a harmful influence on the human body, the environment, and/or nontarget living creatures. For example, a large amount of various types of chemically-synthesized insecticide (chemical pesticide) has been used in order to control insect pests harmful to grass covering the ground in a golf course, thereby causing problems as a source of various types of pollution. Examples of the problems include a problem of labor hygiene for employees of the golf course, a problem of local environmental contamination due to chemical pesticides washed away from the golf course by rainwater and the like, a problem of pesticide contamination of a source of drinking water and the like, and/or a problem of influence on the human body.

In the field of agriculture pertaining to insect pest control, more and more agricultural producers and consumers have recently had crisis awareness about a large number of negative effects of the conventional chemical pesticides. This has triggered a review of an insect pest control method using a new technique instead of chemical pesticides.

Conventionally, it has been known that insects has phototaxis, that insects are easily attracted to light falling within a range of ultraviolet light to blue light, and that light falling within a range of wavelengths longer than the wavelength of yellow light has an effect of repelling insects. Based on these findings, a yellow fluorescent lamp using such characteristics of insects (e.g., see Patent Document 1) and an HID lamp (e.g., see Patent Document 2), or a yellow LED (e.g., see Patent Document 3) has been developed for the purpose of deterring (repelling) insects in orchards and the like.

However, on the other hand, various types of insect catching adhesive tape, colored blue or yellow, which are used for attracting and collecting insect pests have been developed or commercially available (e.g., see Patent Document 4 or 5).

As described above, it has been known that a specific color tone has an effect of attracting or repelling a specific insect (insect pest). However, it is less well known what color tone has what effect on what insect. Furthermore, each of the foregoing conventional techniques is intended to directly exterminate insect pests by using a specific color tone; however, none of them is able to exterminate insect pests very efficiently.

In recent years, various types of biotic control method using microorganisms and/or natural enemy insects as materials for controlling insect pests (i.e., insect pest control methods according to which biotic pesticides using microorganisms and/or natural enemy insects are used as pesticides) have been used. In the field of biotic insect pest control, materials for use in insect pest control are living creatures per se (e.g., microorganisms) or products made thereof. Generally, a material for use in insect pest control has a lethal effect only on a specific insect, and does not exert any influence on other living creatures. Such a biotic pesticide does not contain a compound unlike the conventional chemically-synthesized insecticides, and therefore does not give rise to a new strain of insect pest that has insecticide resistance.

Examples of materials for use in biotic control include natural enemy insects having parasitic and predatory characteristics. In fact, several types of natural enemy insect have already been used in practice. Biotic control techniques using natural enemy insects are roughly classified into biotic control techniques using natural enemy insects introduced from overseas and biotic control techniques using natural enemy insects indigenous to our country. Currently, the biotic control techniques using natural enemy insects introduced from overseas are used.

In recent years, several types of biotic pesticide introduced from overseas have been registered as pesticides, and have started to be used. According to these biotic pesticides, pathogens, natural enemy insects, or the like against insect pests are sprayed so as to prevent the growth of the insect pests. However, the exogenous biotic pesticides are highly likely to affect the ecosystem of an area in which they are used. In view of this, it is hoped to develop insect pest control (i.e., new biotic pesticides) using indigenous natural enemy insects that exert less influence on the ecosystem.

Further, the effects of insect pest control using natural enemy insects cannot be brought about simply by releasing natural enemy insects into fields, and depend on the density of generation of insect pests in the fields and/or the timing of releasing them into the fields.

According to the life history of larval parasitic wasps, they parasitize phytophagous insects in the larval stage, and finally kill their hosts to develop to the adult stage. Such parasitic wasps (most of which are not more than 3 mm in length) use various types of information in searching a wide environment for phytophagous insects that serve as their hosts.

It is known that parasitic wasps search for their hosts by using induced chemicals (volatile odorous substances) emitted from plants damaged by host phytophagous insects. For a specific example, *Cotesia plutellae* search for their hosts by using volatile informational chemicals (odor information) inducively produced by and emitted from crucifers damaged by diamondback moths *(Plutella xylostella).* For another specific example, *Cotesia glomerata* search for their hosts by using volatile informational chemicals (odor information) inductively produced by and emitted from crucifers damaged by white cabbage butterflies *(Pieris rapae).*

It is currently expected that greenhouse farming with less or no pesticides will be practiced by means of control (i.e., biotic control) using natural enemy insects (esp., parasitic wasps), not by means of application of insecticide. However, in order to introduce natural enemy insects at a good timing for the purpose of insect pest control, it is important that desired natural enemy insects be obtained at a desired timing, i.e., that natural enemy insects be bred conveniently.

However, conventionally, it has been difficult to breed parasitic wasps while allowing them to enjoy a long-life span. Since a greenhouse has no food for parasitic wasps, parasitic wasps introduced and released into a greenhouse die one day after the introduction. Further, even when a conventional method according to which food is simply provided in a greenhouse is simply adopted, parasitic wasps cannot easily find the food. This causes such a problem that the percentage of parasitism measured three days after parasitic wasps were introduced into a greenhouse is approximately 20% of the percentage of parasitism measured on the very day they were introduced into the greenhouse. In order to breed parasitic wasps in such a greenhouse, it is necessary to efficiently give food to the parasitic wasps. For that purpose, it is necessary to efficiently attract the parasitic wasps to the food.

The inventors have already filed applications concerning "plant induced indirect defense" by which natural enemy insects are efficiently attracted using odorous components (e.g., see Patent Documents 6 and 7). As described above, a method of efficiently attract parasitic wasps to food by using inductive chemicals that have been identified is very suitable for feeding/breeding of natural enemy insects (e.g., parasitic wasps). However, volatile odorous substances so emitted from plants damaged by insect pests (phytophagous insects) as to attract natural enemy insects are the only known means capable of attracting parasitic wasps, and there has existed no special technique for improving efficiency in insect pest control from a viewpoint of efficiently feeding and breeding natural enemy insects (e.g., parasitic wasps).
[Patent Document 1]
   Japanese Unexamined Patent Publication No. 307054/1999 (*Tokukaihei* 11-307054; published on November 5, 1999)
[Patent Document 2]
   Japanese Unexamined Patent Publication No. 260431/2002 *(Tokukai* 2002-260431; published on September 13, 2002)
[Patent Document 3]
   Japanese Unexamined Patent Publication No. 284482/2003 *(Tokukai* 2003-284482; published on October 7, 2003)
[Patent Document 4]
   Japanese Unexamined Utility Model Specification No. 96788/1983 *(Jitsuzensho* 58-96788; published on July 1, 1983)
[Patent Document 5]
   Japanese Unexamined Patent Publication No. 51909/1996 (*Tokukaihei* 8-51909; published on February 27, 1996)
[Patent Document 6]
   Japanese Unexamined Patent Publication No. 339260/2003 *(Tokukai* 2003-339260; published on December 2, 2003)
[Patent Document 7]
   Japanese Unexamined Patent Publication No. 149420/2004 (*Tokukai* 2004-149420; published on May 27, 2004)

### DISCLOSURE OF INVENTION

The present invention has been made in view of the foregoing problems, and it is an object of the present invention to provide: a feeding method and apparatus for efficiently breeding natural enemy insects that prey on small plant-parasitic insect pests, and for raising the percentage of parasitism thereby; and a rearing method for breeding natural enemy insects. In other words, the object of the present invention is to supply: a feeding method and apparatus for efficiently breeding natural enemy insects such as parasitic wasps (e.g., *Cotesia* plutellae), and for raising the percentage of parasitism thereby; and a rearing method for breeding natural enemy insects.

In order to breed natural enemy insects, a feeding apparatus according to the present invention includes: a food supplying section; a feeding section; a food introducing section via which the food supplying section and the feeding section are in communication with each other; and an attracting section made of a material having a color indicating L* = 20 to 100, a* = -30 to +30, and b* = 20 or more in terms of L*a*b* color system chromaticity.

The feeding apparatus according to the present invention is preferably arranged such that the feeding section is made of fiber, paper, or sponge.

The feeding apparatus according to the present invention is preferably arranged such that the food supplying section is made of glass, polyethylene, polypropylene, or polyethylene terephthalate.

The feeding apparatus according to the present invention is preferably arranged such that the material constituting the attracting section is selected from the group consisting of paper, polyethylene, polypropylene, and polyethylene phthalate.

The feeding apparatus according to the present invention is preferably arranged such that the feeding section and the food introducing section are integrally formed.

The feeding apparatus according to the present invention is preferably arranged such that the natural enemy insects are parasitic wasps.

In order to breed natural enemy insects, a feeding apparatus according to the present invention includes: a food supplying section; a feeding section that is in communication with the food supplying section so that food is supplied from the food supplying section to the feeding section; and an attracting section provided with a material having a color indicating L* = 20 to 100, a* = -30 to +30, and b* = 20 or more in terms of L*a*b* color system chromaticity.

A feeding method according to the present invention includes the step of attracting the natural enemy insects by using a material having a color indicating L* = 20 to 100, a* = -30 to +30, and b* = 20 or more in terms of L*a*b* color system chromaticity.

The feeding method according to the present invention is preferably arranged such that the natural enemy insects are parasitic wasps.

A rearing method according to the present invention for breeding natural enemy insects includes the step of feeding natural enemy insects attracted by using a material having a color indicating L* = 20 to 100, a* = -30 to +30, and b* = 20 or more in terms of L*a*b* color system chromaticity.

A rearing method according to the present invention for breeding natural enemy insects uses a feeding apparatus as described above.

The rearing method according to the present invention is preferably arranged such that the natural enemy insects are parasitic wasps.

The provision of a feeding apparatus according to the present invention in a greenhouse makes it possible to obtain a high survival rate of parasitic wasps and a high percentage of parasitism even three days after the parasitic wasps were introduced into the greenhouse. This makes it possible to establish a farming system that uses less or no pesticides in the greenhouse.

Further, the use of a feeding apparatus according to the present invention, a feeding method according to the present invention, and a rearing method according to the present invention for breeding natural enemy insects makes it possible to dramatically improve efficiency in parasitism by indigenous parasitic wasps and introduced parasitic wasps. This makes it possible to develop sustainable agriculture that puts less stress on the environment.

Additional objects, features, and strengths of the present invention will be made clear by the description below. Further, the advantages of the present invention will be evident from the following explanation in reference to the drawings.

### BRIEF DESCRIPTION OF DRAWINGS

Fig. 1 shows an embodiment of the present invention, and is a perspective view showing a structure of a feeding apparatus according to the present invention.
Fig. 2 shows an embodiment of the present invention, and is a perspective view showing a structure of a feeding apparatus according to the present invention.
Fig. 3 shows an embodiment of the present invention, and is a diagram showing a structure of a feeding apparatus according to the present invention.
Fig. 4 is a graph showing the influence of feeding on the number of days for which adult *Cotesia plutellae* males and females survive.
Fig. 5 shows an embodiment of the present invention, and is a diagram showing a state of feeding conducted by using a feeding apparatus (named *Hachigenki)* according to the present invention in a temperature-controlled room.
Fig. 6 shows an embodiment of the present invention, and is a diagram showing a state of feeding conducted by using a feeding apparatus (named *Hachigenki*) according to the present invention in a small choice chamber.

### BEST MODE FOR CARRYING OUT THE INVENTION

As a result of searching for a rearing method for breeding parasitic wasps, the inventors have found that the problems with the conventional methods can be solved. That is, the present invention relates to a feeding method and apparatus for breeding parasitic wasps (e.g., *Cotesia plutellae),* and to a rearing method for breeding natural enemy insects.

The inventors have found that parasitic wasps are preferably bred by giving honey to them. Specifically, whereas those parasitic wasps which do not ingest honey die in one day, the supply of honey to parasitic wasps raised their survival rate, and made an improvement in the percentage of parasitism.

However, as described above, since a greenhouse has no food for parasitic wasps, parasitic wasps introduced and released into a greenhouse die approximately one day after the introduction. Further, even when a conventional method according to which food is simply provided in a greenhouse is simply adopted, parasitic wasps cannot easily find the food. This causes such a problem that the percentage of parasitism measured three days after parasitic wasps were introduced into a greenhouse is approximately 20% of the percentage of parasitism measured on the very day they were introduced into the greenhouse. In order to breed parasitic wasps in such a greenhouse, it is necessary to efficiently give food to the parasitic wasps. For that purpose, it is necessary to efficiently attract the parasitic wasps to the food.

The inventors have found that the use of an apparatus including a member made of a material having a specific color tone makes it possible to feed parasitic wasps by efficiently attracting them to a source of honey. The inventors have also found that: the use of such an apparatus causes more parasitic wasps to visit a feeding container, thereby making an improvement in their survival rate.

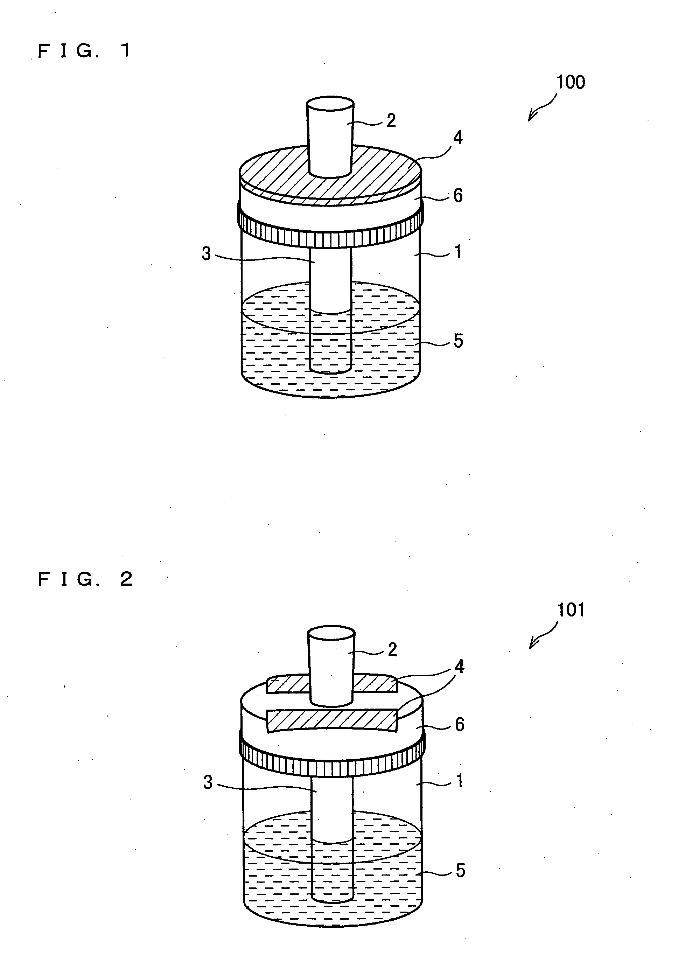

Embodiment 1 of the present invention will be described below with reference to Fig. 1.

As shown in Fig. 1, a feeding apparatus 100 according to the present embodiment includes a food supplying section 1, a feeding section 2, a food introducing section 3 via which the food supplying section 1 and the feeding section 2 are in communication with each other, and an attracting section 4. The food supplying section 1 contains food 5 for feeding natural enemy insects that are to be bred. As shown in Fig. 1, the feeding apparatus 100 according to the present embodiment is integrally constituted by (i) the food supplying section 1 serving as a container containing the food 5 and (ii) a lid 6 provided with the attracting section 4, and the food introducing section 3, which extends from the interior of the food supplying section 1 so as to pass through the attracting section 4, has a top provided with the feeding section 2.

The feeding section 2 is preferably made of fiber, paper, sponge, or the like, and is most preferably made of fiber. Further, the feeding section 2 is preferably a suction wick.

The food introducing section 3 is preferably made of fiber, paper, sponge, or the like, and is most preferably made of fiber. The food introducing section 3 may be made of the same material as is the feeding section 2, the feeding section 2 and the food introducing section 3 may constitute a single member. That is, the feeding section 2 per se may be the food introducing section 3.

The attracting section 4 is made of a material having a color indicating L* = 20 to 100, a* = -30 to +30, and b* = 20 or more in terms of L*a*b* color system chromaticity. Examples of such a material include, but are not limited to, a material selected from the group consisting of organic pigments, inorganic pigments, processed pigments, dyes, and the like. The material is selected from the group consisting of paper, polyethylene, polypropylene, and polyethylene phthalate, and preferably has a color indicating L* = 40 to 80, a* = -20 to +20, and b* = 40 or more in terms of L*a*b* color system chromaticity. As defined in Kojien and other dictionaries, the term "paper" used in this specification is intended to mean a material mainly made of plant fiber.

The food 5 may be appropriately selected depending on the type of natural enemy insect to be bred. Examples of the food 5 include, but are not limited to, honey, sucrose, glucose, fructose, and the like. Most preferably, the food 5 is honey (e.g., honey collected from *Astragalus sinicus,* such honey being hereinafter referred to as "Astragalus honey"). The food 5 preferably has a concentration of 10 to 50%, and more preferably has a concentration of 20 to 50%.

The lid 6 may be made of the same material as is the attracting section 4, and the attracting section 4 and the lid 6 may constitute a single member. That is, the lid 6 per se may be the attracting section 4.

Embodiment 2 of the present invention will be described below with reference to Fig. 2.

As shown in Fig. 2, a feeding apparatus 101 according to the present embodiment includes a food supplying section 1, a feeding section 2, a food introducing section 3 via which the food supplying section 1 and the feeding section 2 are in communication with each other, and an attracting section 4. The food supplying section 1 contains food 5 for feeding natural enemy insects that are to be bred. As shown in Fig. 2, the feeding apparatus 101 according to the present embodiment is integrally constituted by (i) the food supplying section 1 serving as a container containing the food 5 and (ii) a lid 6 provided with the attracting section 4, and the food introducing section 3, which extends from the interior of the food supplying section 1 so as to pass through the attracting section 4, has a top provided with the feeding section 2.

As shown in Fig. 2, Embodiment 2 is arranged such that the attracting section 4 of Embodiment 1 is simply pasted on the lid 6. In the present embodiment, the feeding section 2, the food introducing section 3, and the food 5 can be the same as those of Embodiment 1.

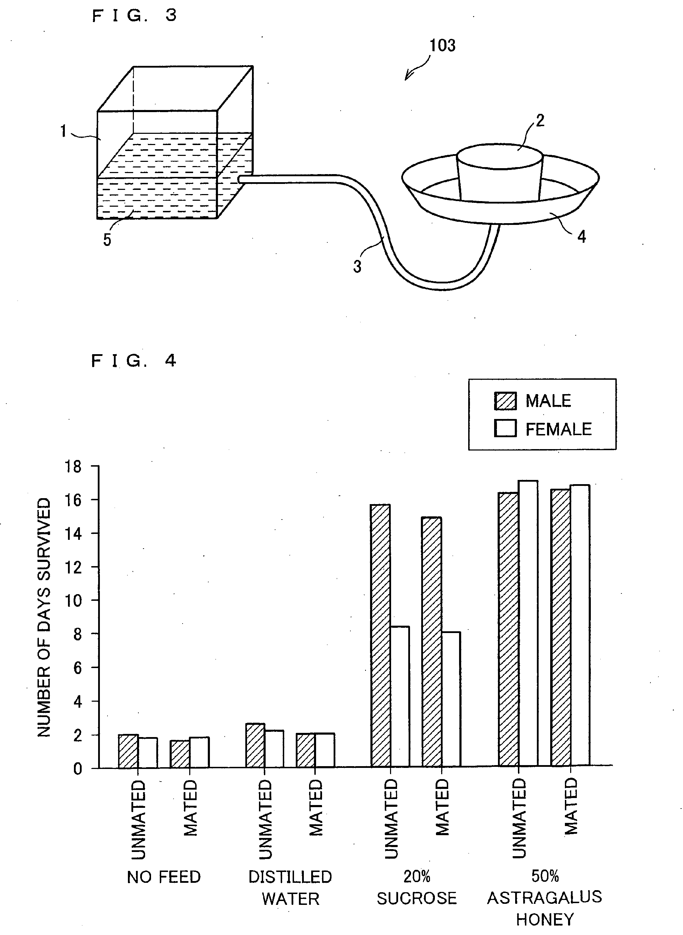

Embodiment 3 of the present invention will be described below with reference to Fig. 3.

As shown in Fig. 3, a feeding apparatus 102 according to the present embodiment includes a food supplying section 1, a feeding section 2, a food introducing section 3 via which the food supplying section 1 and the feeding section 2 are in communication with each other, and an attracting section 4. The food supplying section 1 contains food 5 for feeding natural enemy insects that are to be bred. As shown in Fig. 3, the feeding apparatus 102 according to the present embodiment is arranged such that the interior of the food supplying section 1 serving as a container containing the food 5 and the feeding section 2 provided on the attracting section 4 are connected by the food introducing section 3.

As shown in Fig. 3, the present embodiment is arranged, unlike Embodiments 1 and 2, such that the food supplying section is separated from the feeding section 2 or the attracting section 4.

The feeding section 2 is preferably made of fiber, paper, sponge, or the like, and is most preferably made of fiber.

The food introducing section 3 is preferably made of fiber, paper, sponge, or the like, and is most preferably made of fiber.

The attracting section 4 may be made of a material used in Embodiments 1 and 2.

The food 5 may be appropriately selected depending on the type of natural enemy insect to be bred. Examples of the food 5 include, but are not limited to, honey, sucrose, glucose, fructose, and the like. Most preferably, the food 5 is honey (e.g., Astragalus honey). The food 5 preferably has a concentration of 10 to 50%, and more preferably has a concentration of 20 to 50%.

In Fig. 3, a single food supplying section 1 and a single feeding section 2 are connected by a single food introducing section 3. However, it is possible that a single food supplying section 1 and a plurality of feeding sections 2 are connected by a plurality of food introducing sections 3.

Further, in Fig. 3, in cases where the feeding section 2 is made of a material capable of retaining a sufficient amount of food, the food supplying section 1 and the food introducing section 3 can be omitted by using the feeding section 2 also as the food supplying section 1 and the food introducing section 3. In this case, the feeding apparatus 102 is constituted by the feeding section 2 and the attracting section 4.

The phrase "color indicating L* = 20 to 100, a* = -30 to +30, and b* = 20 or more in terms of L*a*b* color system chromaticity" used in this specification is intended to mean a color tone ranging from yellow green to orange through yellow.

Thus, it can be said that a feeding apparatus according to the present invention only needs to include at least a feeding section and an attracting section that is made of a material having a specific color tone. That is, it is to be noted that a feeding apparatus including a food supplying section and a food introducing section each having a structure other than those described in the foregoing embodiments is also encompassed in the technical scope of the present invention.

A feeding method according to the present invention for breeding natural enemy insects only needs to include a step of attracting natural enemy insects by using a material having a color indicating L* = 20 to 100, a* = -30 to +30, and b* = 20 or more in terms of L*a*b* color system chromaticity, and is not particularly limited in terms of other specific arrangements.

Examples of the material having a color indicating L* = 20 to 100, a* = -30 to +30, and b* = 20 or more in terms of L*a*b* color system chromaticity include, but are not limited to, a material selected from the group consisting of organic pigments, inorganic pigments, processed pigments, dyes, and the like. Further, the material may be a material, having color indicating L* = 40 to 80, a* = -20 to +20, and b* = 40 or more in terms of L*a*b* color system chromaticity, which is selected from the group consisting of paper, polyethylene, polypropylene, and polyethylene phthalate.

A rearing method according to the present invention for breeding natural enemy insects only needs to use the aforementioned feeding method, and is not particularly limited in terms of other specific arrangements. Specifically, the rearing method according to the present invention for breeding natural enemy insects only needs to include a step of feeding natural enemy attracted by using a material having a color indicating L* = 20 to 100, a* = -30 to +30, and b* = 20 or more in terms of L*a*b* color system chromaticity.

Examples of the material having a color indicating L* = 20 to 100, a* = -30 to +30, and b* = 20 or more in terms of L*a*b* color system chromaticity include the same materials as those described in the aforementioned feeding method.

Preferably, the rearing method according to the present invention for breeding natural enemy insects specifically uses the aforementioned feeding apparatus.

It is easily understood by a person skilled in the art that: the color tone and/or food depend on the type of natural enemy insect to be bred, and therefore can be selected depending on the type of target natural enemy insect.

That is, the object of the present invention is to provide a feeding method and apparatus for breeding natural enemy insects and a rearing method for breeding natural enemy insects, and does not depend on the shape of each of the members specifically described in this specification. Therefore, it is to be noted that a feeding apparatus having a shape other than those described in the foregoing embodiments is also encompassed in the scope of the present invention.

The present invention will be described more in detail by way of the following examples, but is not limited to these examples.

### [Examples]

### (Example 1: Influence of Feeding on the Number of Days for Which Parasitic Wasps Survive)

Fig. 4 shows the influence of feeding on the number of days for which adult Cotesia plutellae males and females survive. Under such circumstances that no food is given (see "NO FOOD" or "DISTILLED WATER" in the figure), there is not a difference between the number of days for which unmated wasps survived and the number of days for which mated wasps survived, nor is there a difference between the number of days for which male wasps survived and the number of days for which female wasps survived. However, the supply of 20% sucrose allowed males to survive two weeks or longer, and females to survive one week or longer. Furthermore, the supply of 50% Astragalus honey allowed both males and females to survive two weeks or longer. Thus, it was found that food is a key to the survival of *Cotesia plutellae* and that *Cotesia plutellae* can only survive up to one day without food.

### (Example 2: Change in the Percentage of Parasitism by Parasitic Wasps due to a Difference between Feeding Apparatuses)

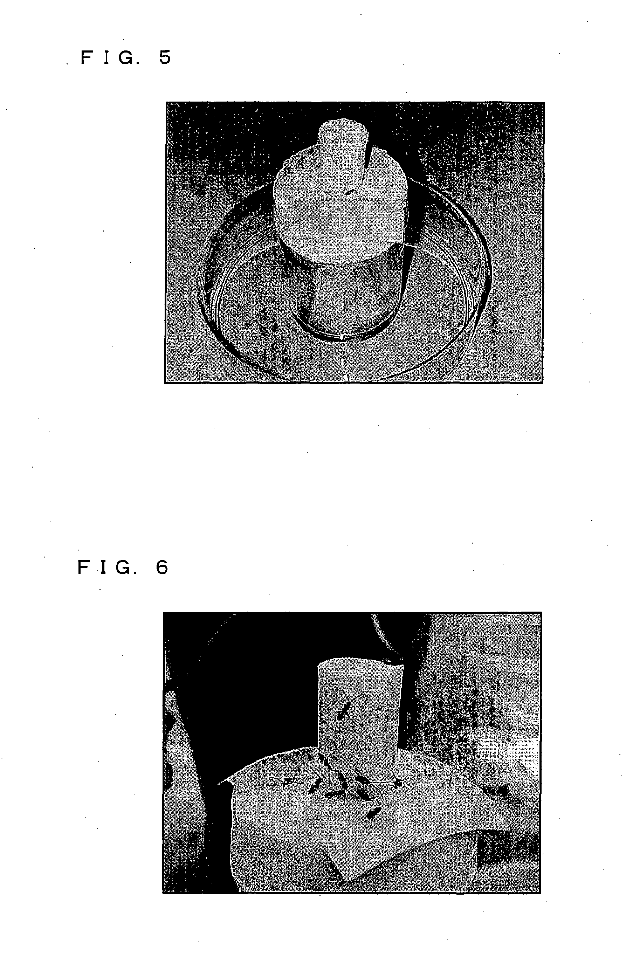

A feeding apparatus (named *Hachigenki)* shaped as shown in Fig. 5 was used to examine whether or not parasitic wasps can be efficiently fed in a relatively wide space such as a greenhouse. A total of 25 feeding apparatuses (named *Hachigenki*) were provided in a greenhouse with the dimensions 7m × 7m × 3m so as to be placed 5 × 5 at intervals of 30 cm. A total of 100 second-instar diamondback moth larvae were diagonally released by 20 in the greenhouse, and a total of 10 *Cotesia plutellae* (females) were released into the greenhouse. The percentage of parasitism measured one day after the release was compared with the percentage of parasitism measured three days after the release.

The percentages of *Cotesia plutellae,* fed with 50% Astragalus honey, which had parasitized the diamondback moths were measured in cases where a plastic plate having a tone of white was used as an attracting section 4 of a feeding apparatus (named *Hachigenki)* and where a plastic plate having a tone of yellow was used as an attracting section 4 of a feeding apparatus (named *Hachigenki*)*.*

**[Table 1]**

| | Percentage of Parasitism Measured One Day after the Release | Percentage of Parasitism Measured Three Days after the Release |
|---|---|---|
| White | 89.2 | 24.5 |
| Yellow | 91.7 | 84.6 |

As shown in Example 1, *Cotesia plutellae* can survive approximately one day even without food. As a result, the percentage of parasitism measured one day after the release is high regardless of the color of an attracting section of a feeding apparatus (named *Hachigenki*)*.* However, there was a change in the percentage of parasitism measured three days after the release, and this change is due to the color of an attracting section of a feeding apparatus (named *Hachigenki).* Specifically, there was a sharp decrease in the percentage of parasitism in cases where the attracting section of the feeding apparatus (named *Hachigenki)* had a tone of white; however, the percentage of parasitism was kept high in cases where he attracting section of the feeding apparatus (named *Hachigenki*) had a tone of yellow.

As described above, it was found that parasitic wasps dies in one day without food, that the supply of food raises their survival rate and makes an improvement in the percentage of parasitism, and that food is efficiently supplied by using a feeding apparatus (named *Hachigenki*) having a yellow attracting section.

### (Example 3: Change in the Percentage of Parasitism due to the Location in Which a Feeding Apparatus Is Placed)

A study was conducted to examine at what height a feeding apparatus (named *Hachiganki)* is preferably placed. Specifically, the percentages of parasitism by parasitic wasps were measured in cases where a feeding apparatus (named *Hachigenki)* shown in Fig. 5 was placed at a height of 50 cm above the ground and where it was placed at a height of 2 m above the ground.

**[Table 2]**

| | Percentage of Parasitism Measured One Day after the Release | Percentage of Parasitism Measured Three Days after the Release |
|---|---|---|
| 2 m above the Ground | 78.5 | 11.2 |
| 50 cm above the Ground | 86.3 | 79.8 |

As shown in Table 2, it was found that the feeding apparatus (named *Hachigenki)* is preferably placed at a height of 50 cm above the ground.

The embodiments and concrete examples of implementation discussed in the foregoing detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

### INDUSTRIAL APPLICABILITY

The provision of a feeding apparatus according to the present invention in a greenhouse makes it possible to establish a farming system that uses less or no pesticides in the greenhouse. Further, the use of a feeding apparatus according to the present invention, a feeding method according to the present invention, and a rearing method according to the present invention for breeding natural enemy insects makes it possible to dramatically improve efficiency in parasitism by indigenous parasitic wasps and introduced parasitic wasps. This makes it possible to develop sustainable agriculture that puts less stress on the environment.

## Claims

1. A feeding apparatus for breeding natural enemy insects, the feeding apparatus comprising:
a food supplying section;
a feeding section;
a food introducing section via which the food supplying section and the feeding section are in communication with each other; and
an attracting section made of a material having a color indicating L* = 20 to 100, a* = -30 to +30, and b* = 20 or more in terms of L*a*b* color system chromaticity.

2. The feeding apparatus as set forth in claim 1, wherein the feeding section is made of fiber, paper, or sponge.

3. The feeding apparatus as set forth in claim 1, wherein the food supplying section is made of glass, polyethylene, polypropylene, or polyethylene terephthalate.

4. The feeding apparatus as set forth in claim 1, wherein the material constituting the attracting section is selected from the group consisting of paper, polyethylene, polypropylene, and polyethylene phthalate.

5. The feeding apparatus as set forth in claim 1, wherein the feeding section and the food introducing section are integrally formed.

6. The feeding apparatus as set forth in claim 1, wherein the natural enemy insects are parasitic wasps.

7. A feeding apparatus for breeding natural enemy insects, the feeding apparatus comprising:
a food supplying section;
a feeding section that is in communication with the food supplying section so that food is supplied from the food supplying section to the feeding section; and
an attracting section provided with a material having a color indicating L* = 20 to 100, a* = -30 to +30, and b* = 20 or more in terms of L*a*b* color system chromaticity.

8. A feeding method for breeding natural enemy insects, the feeding method comprising the step of attracting the natural enemy insects by using a material having a color indicating L* = 20 to 100, a* = -30 to +30, and b* = 20 or more in terms of L*a*b* color system chromaticity.

9. The feeding method as set forth in claim 8, wherein the natural enemy insects are parasitic wasps.

10. A rearing method for breeding natural enemy insects, the rearing method comprising the step of feeding natural enemy insects attracted by using a material having a color indicating L* = 20 to 100, a* = -30 to +30, and b* = 20 or more in terms of L*a*b* color system chromaticity.

11. A rearing method for breeding natural enemy insects, the rearing method using a feeding apparatus as set forth in any one of claims 1 to 7.

12. The rearing method as set forth in claim 10 or 11, wherein the natural enemy insects are parasitic wasps.
